# EUROPÄISCHE PATENTSCHRIFT

(11) **EP 1 446 661 B1**
(45) Veröffentlichungstag und Bekanntmachung des Hinweises auf die Patenterteilung: **14.04.2010**
(21) Anmeldenummer: 02801852.1
(22) Anmeldetag: 22.10.2002
(51) Int. Cl.: G01N 27/407, G01N 33/00

(54) **SENSORELEMENT UND VERFAHREN ZUR BESTIMMUNG VON STICKSTOFFOXIDEN**
SENSOR ELEMENT AND METHOD FOR DETERMINING NITROGEN OXIDES
ELEMENT CAPTEUR ET PROCEDE DE DETECTION D'OXYDES D'AZOTE

(30) Priorität: 23.10.2001 DE 10152176
(43) Veröffentlichungstag der Anmeldung: 18.08.2004
(73) Patentinhaber: ROBERT BOSCH GMBH, 70442 Stuttgart (DE)
(72) Erfinder: BRUEGGEN, Gerhard, 72181 Starzbach (DE); GRUENWALD, Werner, 70839 Gerlingen (DE)
(86) Internationale Anmeldenummer: PCT/DE2002/003974
(87) Internationale Veröffentlichungsnummer: WO 2003/036282

(56) Entgegenhaltungen:
- EP-A- 0 807 818
- WO-A-01/02845
- US-A- 4 770 760
- US-A- 4 927 517
- PATENT ABSTRACTS OF JAPAN vol. 013, no. 228 (P-877), 26. Mai 1989 (1989-05-26) & JP 01 039545 A (NISSAN MOTOR CO LTD), 9. Februar 1989 (1989-02-09)

## Beschreibung

Die Erfindung betrifft ein Sensorelement eines Gassensors und ein Verfahren zur Messung einer Gaskomponente in einem Gasgemisch nach dem Oberbegriff der unabhängigen Ansprüche..

### Stand der Technik

Vor allem in Abgasen von Verbrennungsmotoren liegen oft kleine Schadstoffmengen neben einem hohen Gehalt an Sauerstoff vor. Dies führt insbesondere bei der Bestimmung der Stickoxidkonzentration in Abgasen zu Problemen, da der hohe Sauerstoffgehalt die indirekte Bestimmung der Stickoxide, bei der die Stickoxide elektrochemisch zersetzt und die Menge des dabei freigesetzten Sauerstoffs bestimmt wird, erschwert.

Aus der EP 769 694 A1 ist ein elektrochemisches Sensorelement eines Gassensors zur Bestimmung der NOₓ-Konzentration in einem Gasgemisch bekannt, bei dem innerhalb des Sensorelements zunächst elektrochemisch ein Großteil des Sauerstoffs entfernt und eine vorbestimmte, konstant niedrige Sauerstoffkonzentration eingestellt wird, bevor die Bestimmung des Stickoxidgehalts erfolgt. Die selektive Entfernung des Sauerstoffs erfordert jedoch zum einen spezielle, katakatalytisch inaktive Elektrodenmaterialien und zum anderen eine exakte Steuerung der entsprechenden elektrochemischen Pumpzellen. Das beschriebene Sensorelement birgt daher die Gefahr der Störanfälligkeit und bedingt eine kostenintensive Herstellung.

Weiterhin ist aus der WO 01/02845 A bekannt, einen elektrochemischen Gassensor, insbesondere zur Bestimmung von NOx in Abgasen von Verbrennungsmotoren mit zwei zueinander parallel angeordneten Messgasräumen auszustatten, wobei in den betreffenden Messgasräumen jeweils amperometrische Pumpzellen vorgesehen sind.

Aufgabe der vorliegenden Erfindung ist es, ein Sensorelement eines Gassensor bereitzustellen, das die Bestimmung von geringen Schadstoffmengen zuverlässig ermöglicht und einfach aufgebaut ist.

### Vorteile der Erfindung

Das erfindungsgemäße Sensorelement bzw. das erfindungsgemäße Verfahren mit den kennzeichnenden Merkmalen der unabhängigen Ansprüche löst in vorteilhafter Weise die der Erfindung zugrundeliegende Aufgabe. Das erfindungsgemäße Sensorelement weist zwei nach dem Nernstprinzip arbeitende potentiometrische Messzellen auf. Da beiden Messzellen ein im wesentlichen gleicher Diffusionswiderstand gegenüber dem Messgas vorangestellt ist und beide Messzellen nach dem gleichen Messprinzip betrieben werden, und Unterschiede in den Polarisationswiderständen durch Anlegen eines Konstantsroms ausgeglichen werden, Rann man ein der Konzentration der zu bestimmenden Gaskomponenten proportionales Messsignal durch einfache Differenzbildung der Messsignale beider Messzellen mit hoher Genauigkeit erhält.

Durch die in den Unteransprüchen aufgeführten Maßnahmen sind weitere vorteilhafte Weiterbildungen und Verbesserungen des im Hauptanspruch angegebenen Sensorelements möglich. So wirkt es sich positiv auf die Genauigkeit des Sensorelements aus, wenn die innerhalb des Sensorelements dem Messgas ausgesetzten Elektroden der Messzellen in parallel angeordneten Gasräumen vorgesehen sind und unter weitgehend gleichen Temperaturbedingungen betrieben werden.

Weiterhin ist es vorteilhaft, wenn im Sensorelement zusätzlich eine elektrochemische Pumpzelle vorgesehen ist, die den Meßzellen in Strömungsrichtung des Meßgases vorangestellt ist und der Verringerung des Sauerstoffgehalts im Meßgas dient. Da die Pumpzelle mit einer konstanten Pumpspannung betrieben werden kann, wird eine vorteilhaft einfache Betriebsweise des Sensorelements ermöglicht.

Erfindungsgemäß werden die Elektroden der beiden potentiometrischen Meßzellen mit einem Konstantstrom beaufschlagt, wobei dessen Größe jeweils so eingestellt wird, daß Unterschiede in den Polarisationswiderständen der Elektroden ausgeglichen werden können.

### Zeichnung

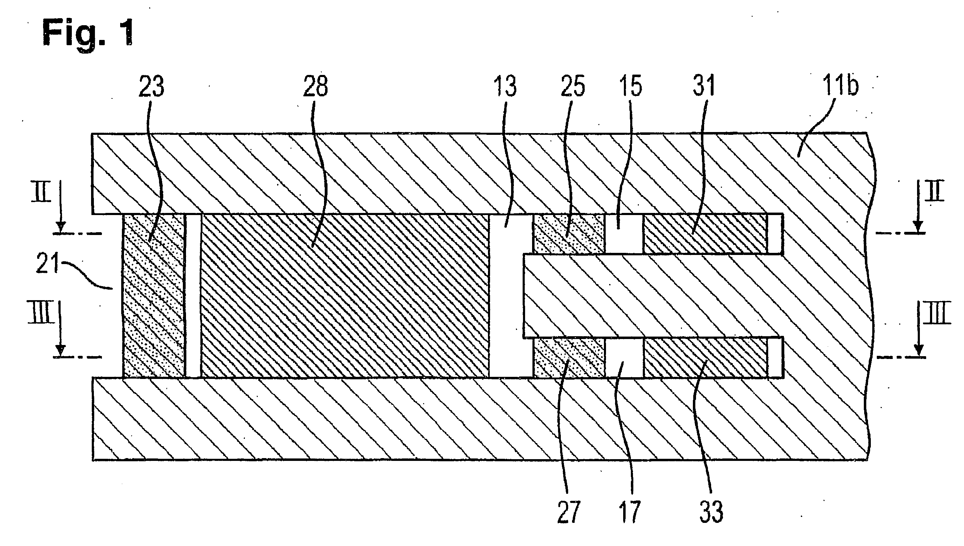

Zwei Ausführungsbeispiele der Erfindung sind in der Zeichnung dargestellt und in der nachfolgenden Beschreibung näher erläutert. Es zeigt Figur 1 einen Querschnitt durch die Großfläche eines Sensorelements gemäß einem ersten Ausführungsbeispiel der vorliegenden Erfindung.

### Ausführungsbeispiele

Die Figur 1 zeigt einen prinzipiellen Aufbau einer ersten. Ausführungsform der vorliegenden Erfindung. Mit 10 ist ein planares Sensorelement eines elektrochemischen Gassensors bezeichnet, das beispielsweise eine Mehrzahl von sauerstoffionenleitenden Festelektrolytschichten 11a, 11b, 11c, 11d, 11e und 11f aufweist. Die Festelektrolytschichten 11a-11f werden dabei als keramische Folien ausgeführt und bilden einen planaren keramischen Körper. Die integrierte Form des planaren keramischen Körpers des Sensorelements 10 wird durch Zusammenlaminieren der mit Funktionsschichten bedruckten keramischen Folien und anschließendem Sintern der laminierten Struktur in an sich bekannter Weise hergestellt. Jede der Festelektrolytschichten 11a-11f ist aus sauerstoffionenleitendem Festelektrolytmaterial, wie beispielsweise mit Y₂O₃ teil- oder vollstabilisiertem ZrO₂ ausgeführt.

Das Sensorelement 10 beinhaltet einen ersten inneren Gasraum 13 und zwei weitere innere Gasräume 15 und 17, wobei alle drei inneren Gasräume vorzugsweise in derselben Schichtebene 11b ausgebildet sind und beispielsweise eine lichte Höhe von 2 µm aufweisen. Die beiden weiteren inneren Gasräume 15, 17 liegen bevorzugt parallel nebeneinander und erstrecken sich ausgehend vom ersten inneren Gasraum 13 jeweils kanalförmig. Unabhängig von den inneren Gasräumen 13, 15 und 17 ist beispielsweise in einer weiteren Schichtebene 11d ein Referenzgaskanal 19 angeordnet, der an einem Ende aus dem planaren Körper des Sensorelements 10 herausführt und mit einer Referenzatmosphäre wie beispielsweise Luft in Verbindung steht.

Das Sensorelement 10 hat ferner eine Gaseintrittsöffnung 21, die das Meßgas in den ersten inneren Gasraum 13 leitet. Die Gaseintrittsöffnung 21 ist beispielsweise in derselben Schicht 11b wie die Meßzellen 13, 15 und 17 angeordnet und weist beispielsweise eine lichte Höhe von 1 µm auf. Am Eingang zum ersten inneren Gasraum 13 ist hinter der Gaseintrittsöffnung 21 in Diffusionsrichtung des Meßgases eine erste Diffusionsbarriere 23 beispielsweise aus porösem keramischem Material ausgebildet. Zwischen den inneren Gasräumen 13 und 15 sowie zwischen den inneren Gasräumen 13 und 17 befindet sich in Diffusionsrichtung des Meßgases je eine weitere Diffusionsbarriere 25 und 27.

Im ersten inneren Gasraum 13 ist eine erste innere Elektrode 28 angeordnet, die eine Länge von bis zu 6 mm aufweisen kann. Die innere Elektrode 28 bildet mit einer im Referenzgaskanal angeordneten äußeren Elektrode eine elektrochemische Pumpzelle. Im zweiten und dritten inneren Gasraum 15, 17 befindet sich je eine weitere innere Elektrode 31, 33. Die dazugehörige äußere Elektroden 35 befindet sich vorzugsweise auf einer dem Meßgas direkt ausgesetzten Großfläche des Sensorelements. Die äußere Elektrode 35 kann in einfacher oder doppelter Ausführung vorgesehen sein.

Um zu gewährleisten, daß an den inneren Elektroden 28, 31 in den inneren Gasräumen 13, 15 keine Zersetzung von Gaskomponenten auftritt, bestehen die dort angeordneten inneren Elektroden 28, 31 beispielsweise aus Gold oder einer Gold/Platin-Legierung. Im inneren Gasraum 17 wird dagegen als Elektrode 33 ein katalytisch aktives Material verwendet, das beispielsweise die katalytische Zersetzung von NOₓ in Sauerstoff und Stickstoff bewerkstelligen kann. Hierfür eignet sich beispielsweise Rhodium oder eine Platin/Rhodium-Legierung. Die äußeren Elektroden bestehen vorzugsweise aus einem katalytisch aktiven Material, beispielsweise aus Platin. Das Elektrodenmaterial für alle Elektroden wird dabei in an sich bekannter Weise als Cermet eingesetzt, um mit den keramischen Folien zu versintern.

In den keramischen Grundkörper des Sensorelements 10 ist ferner zwischen zwei hier nicht dargestellten elektrischen Isolationsschichten ein Widerstandsheizer eingebettet. Der Widerstandsheizer dient dem Aufheizen des Sensorelements 10 auf die notwendige Betriebstemperatur. Dabei liegt an den räumlich eng benachbarten Elektroden 31, 33 im wesentlichen die gleiche Temperatur vor.

Während des Betriebs des Sensorelements wird durch einen Sauerstofftransfer zwischen den Elektroden der Pumpzelle im ersten inneren Gasraum 13 durch Zu- oder Abpumpen von Sauerstoff ein niedriger Sauerstoffpartialdruck (beispielweise 1000 ppm) eingestellt. Die auf einen niedrigen Sauerstoffpartialdruck eingestellte Meßatmosphäre im inneren Gasraum 13 gelangt nun über die Diffusionsbarrieren 25 und 27 in die inneren Gasräume 15 und 17. Im zweiten inneren Gasraum 15 befindet sich die zweite innere Elektrode 31, die zusammen mit der äußeren Elektrode als erste Konzentrations- oder Nernstzelle betrieben wird.

Unter einer elektrochemischen Nernst- oder Konzentrationszelle wird eine Zweielektrodenanordnung verstanden, deren Elektroden unterschiedlichen Konzentrationen eines zu bestimmenden Gases ausgesetzt sind. Dabei resultiert gemäß der Nernst'schen Gleichung eine meßbare Potentialdifferenz zwischen den Elektroden der Konzentrationszelle.

Die erste Konzentrationszelle dient der Bestimmung des Sauerstoffgehalts innerhalb des Sensorelements. Durch die Wahl des entsprechenden Sauerstoffpartialdrucks bzw. des Elektrodenmaterials wird sichergestellt, daß keine katalytische Zersetzung der zu bestimmenden Gaskomponente an der zweiten inneren Elektrode 31 stattfindet.

Im inneren Gasraum 17 ist die dritte innere Elektrode 33 angeordnet, die zusammen mit der äußeren Elektrode als zweite Konzentrations- oder Nernstzelle betrieben wird. Dabei wird aufgrund des katalytisch aktiven Materials der dritten inneren Elektrode 33 die zu bestimmende Gaskomponente zersetzt. Während beispielsweise Stickoxide dabei Sauerstoff freisetzen, wird bei der Zersetzung von Kohlenwasserstoffen Sauerstoff verbraucht.

Dabei resultiert an der dritten inneren Elektrode 33 eine pro portional zum Betrag an zersetzter Gaskomponente erhöhte bzw. verringerte Sauerstoffkonzentration. Das an der dritten inneren Elektrode sich einstellende Potential wird als Potentialdifferenz gegenüber dem Potential der äußeren Elektrode gemessen. Durch einfache Differenzbildung der an den Elektroden der ersten und zweiten Konzentrationszelle anliegenden Spannungen erhält man ein der Konzentration der zu bestimmenden Gaskomponente proportionales Meßsignal.

Die zweite und dritte innere Elektrode 31, 33 bestehen aus unterschiedlichen Elektrodenmaterialien. Dabei ist nicht; anzuschließen, daß die inneren Elektroden 31, 33 unterschiedlich polarisiert werden und die an der ersten bzw. zweiten Konzentrationszelle gemessenen Spannungen eine Abhängigkeit vom gewählten Elektrodenmaterial zeigen. Dies Kann zu einer Verfälschung der Meßsignale führen. Um dies zu vermeiden, wird erfindungsgemäß an die Elektroden der ersten bzw. zweiten Konzentrationszelle ein Konstantstrom angelegt, dessen Höhe für jede Konzentrationszelle so gewählt wird, daß auftretende Polarisationswiderstände ausgeglichen werden. Die Höhe des Konstantstroms kann in einem Vorversuch in einer Gasatmosphäre, die die zu bestimmende Gaskomponente nicht enthält, so eingestellt werden, daß die Differenz der mittels der ersten und zweiten Konzentrationszelle gemessenen Spannungen gegen 0 geht und gegebenenfalls als Kennlinie hinterlegt werden. Ein weiterer Vorteil eines angelegten Konstantstromes ist darin zu sehen, daß die katalytische Aktivität der dritten inneren Elektrode 33 gesteigert wird. Dies ist Folge des Ab - bzw, Zupumpens von Sauerstoff zur Oberfläche der dritten inneren Elektrode 33.

Die an die Elektroden der Pumpzelle anzulegende Pumpspannung wird auf einen konstanten Wert eingestellt, der einerseits ausreichend hoch ist, den überwiegenden Teil des im Gasgemisch vorhandenen Sauerstoffs zu entfernen bzw. bei sauerstoffarmen Gemischen Sauerstoff ausreichend zuzuführen, andererseits nicht zur elektrochemischen Zersetzung der zu bestimmenden Gaskomponente an der inneren Elektrode 28 führt. So ist beispielsweise bei Temperaturen von 600 - 800°C eine Pumpspannung von ca. 400 - 500 mV, vorzugsweise 450 mV ausreichend, um im inneren Gasraum 13 einen Sauerstoffpartialdruck von 10⁻⁵ bar einzustellen.

Eine weitere Ausführungsform der Erfindung besteht darin, die zwischen dem inneren Gasraum 13 einerseits und den weiteren inneren Gasräumen 15, 17 andererseits angeordneten Diffusionsbarrieren 25, 27 wegzulassen und anstatt dessen die innere Elektrode 28 so zu dimensionieren, daß an deren den inneren Gasräumen 15, 17 zugewandter Seite eine ausreichend niedrige und homogene Sauerstoffkonzentration vorliegt. Dabei kann die Diffusionsstrecke zwischen erster innerer Elektrode 28 und zweiter bzw. dritter innerer Elektrode 31, 33 auf < 200 µm verkürzt werden.

Bei einem weiteren Ausführungsbeispiel der vorliegenden Erfindung ist die äußere Elektrode 35 nicht auf einer Großfläche des Sensorelements angeordnet, sondern im Referenzgaskanal. Ein Vorteil dieser Variante ist das konstante Referenzpotential, das sich dabei an der äußeren Elektrode einstellt, sowie eine vereinfachte Herstellung des Sensorelements, da die äußeren Elektroden in einem Druckschritt erzeugt werden können.

Die bereits im Rahmen des ersten Ausführungsbeispiels beschriebenen Betriebsweisen der Pumpzelle bzw. der Konzentrationszellen sind auch bei einem Sensorelement gemäß dem zweiten Ausführungsbeispiel realisierbar. Darüber hinaus ermöglicht die Anordnung der äußeren Elektrode im Referenzgaskanal eine dynamische Regelung der an die Elektroden der Pumpzelle anzulegenden Spannung. So kann beispielsweise das Meßsignal der ersten Konzentrationszelle zur Steuerung herangezogen werden, indem die Pumpspannung so variiert wird, daß eine konstant niedrige Sauerstoffkonzentration an der zweiten inneren Elektrode 31 der ersten Konzentrationszelle registriert wird.

Die Anwendungsmöglichkeiten des erfindungsgemäßen Sensorelements sind beispielsweise in der Schadstoffdetektion in Abgasen von Verbrennungsmotoren zu sehen. So ermöglicht insbesondere die Detektion von Stickoxiden eine Kontrolle beispielsweise der Funktionstüchtigkeit oder des Beladungszustands eines NOx-Speicherkatalysators. Dazu wird das Sensorelement in einem Abgasstrang dem NOx-Speicherkatalysator in Strömungsrichtung des Abgases nachgeordnet angebracht. Darüber hinaus wird durch die Bestimmung von Ammoniak die Kontrolle von mit Ammoniak oder Harnstoff betriebenen SCR-Systemen ermöglicht. Dabei wird das Sensorelement im Abgasstrang zwischen Abgasnachbehandlungseinheit und Auspuff angeordnet und der Ammoniakgehalt des austretenden Abgases kontrolliert.

Auch bei Verbrennungsanlagen zu Heizzwecken kann das Sensorelement zur Schadstoffanalyse verwendet werden. Darüber hinaus besteht die Möglichkeit, durch die Detektion beispielsweise von Methan die Vollständigkeit der Verbrennung zu überprüfen. Grundsätzlich ermöglicht das Sensorelement sowohl den rein qualitativen Nachweis der Existenz einer zu messenden Gaskomponente als auch die Bestimmung von deren Konzentration in einem Gasgemisch.

Weitere vorteilhafte Ausführungsformen der vorliegenden Erfindung sind Gegenstand der Patentansprüche.

## Patentansprüche

1. Gassensor zur Bestimmung von Gaskomponenten in Gasgemischen, insbesondere von NOx in Abgasen von Verbrennungsmotoren, enthaltend ein Sensorelement (10), mit einem ersten inneren Gasraum (13,21) und zwei weiteren inneren Gasräumen (15,17), wobei sich die weiteren inneren Gasräume (15,17) ausgehend von dem ersten inneren Gasraum (13,21) jeweils kanalförmig erstrecken und mit einer ersten elektrochemischen Konzentrationszelle zur Bestimmung von freiem Sauerstoff im Gasgemisch, die eine erste Elektrode (31) aufweist, die sich in einem der weiteren inneren Gasräume (15) befindet sowie eine zweite Elektrode aufweist, mit einer zweiten elektrochemischen Konzentrationszelle zur Bestimmung der Summe aus freiem Sauerstoff und aus der Zersetzung der zu bestimmenden Gaskomponente resultierendem oder dabei verbrauchtem Sauerstoff, die eine weitere erste Elektrode (33) aufweist, die sich in dem anderen des weiteren inneren Gasraum (17) befindet sowie eine weitere zweite, katalytisch aktive Elektrode aufweist, wobei der ersten Elektrode (31) der ersten Konzentrationszelle ein Diffusionswiderstand in Strömungsrichtung des Gasgemisches vorangestellt ist, der im Wesentlichen gleich einem der ersten Elektrode (33) der zweiten Konzentrationszelle vorangestellten Diffusionswiderstand ist, **dadurch gekennzeichnet, dass** der Gassensor ein Mittel zur Bildung der Differenz der *potentiometrischen* Signale der ersten und der zweiten Konzentrationszelle umfasst sowie ein weiteres Mittel zum Anlegen eines Konstantstroms an die Elektroden mindestens einer der elektrochemischen Konzentrationszellen, wobei der Konstantstrom so eingestellt ist, dass Unterschiede in den Polarisationswiderständen der potentiometrischen Elektroden ausgeglichen werden.

2. Gassensor nach Anspruch 1, **dadurch gekennzeichnet, dass** eine Gaseintrittsöffnung (21) für das Gasgemisch in das Innere des Sensorelements vorgesehen ist, und dass die ersten Elektroden (31, 33) in einer Schichtebene (11 b) des Sensorelements durch eine im Wesentlichen gleiche Diffusionsstrecke von der Gaseintrittsöffnung (21) entfernt angeordnet sind.

3. Gassensor nach Anspruch 1 oder 2, **dadurch gekennzeichnet, dass** die weiteren inneren Gasräume (15, 17) in einer Schichtebene (11b) parallel nebeneinander angeordnet sind.

4. Gassensor nach Anspruch 3, **dadurch gekennzeichnet, dass** in Strömungsrichtung des Gasgemisches zwischen der Gaseintrittsöffnung (21) und den weiteren inneren Gasräumen (15, 17) der erste innere Gasraum (13) ausgebildet ist, in dem sich eine innere Pumpelektrode (28) einer elektrochemischen Pumpzelle befindet.

5. Gassensor nach Anspruch 4, **dadurch gekennzeichnet, dass** an die Elektroden (28, 29) der elektrochemischen Pumpzelle eine konstante Spannung angelegt wird.

6. Gassensor nach Anspruch 4 oder 5, **dadurch gekennzeichnet, dass** eine äußere Pumpelektrode (29) der elektrochemischen Pumpzelle in einem mit Referenzgas in Kontakt stehenden inneren Gasraum (19) angeordnet ist.

7. Gassensor nach einem der vorhergehenden Ansprüche, **dadurch gekennzeichnet, dass** die zweite Elektrode (35) dem Gasgemisch ausgesetzt ist.

8. Gassensor nach einem der vorhergehenden Ansprüche, **dadurch gekennzeichnet, dass** beide Konzentrationszellen räumlich so angeordnet sind, dass beide vergleichbaren Temperaturbedingungen ausgesetzt sind.

9. Verfahren zur Bestimmung von Gaskomponenten in Gasgemischen mit einem Gassensor nach einem der vorhergehenden Ansprüche, wobei mittels der ersten elektrochemischen Konzentrationszelle des im Gassensor enthaltenen Sensorelements die Konzentration von freiem Sauerstoff und mittels der zweiten elektrochemischen Konzentrationszelle des Sensorelements die Summe von freiem Sauerstoff und mindestens einer zweiten Gaskomponente bestimmt wird, **dadurch gekennzeichnet, dass** ein Messsignal durch die Differenz der potentiometrischen Signale der beiden elektrochemischen Konzentrationszellen gebildet wird und dass an die Elektroden mindestens einer der elektrochemischen Konzentrationszellen ein konstanter Strom angelegt wird wobei die Größe des Konstantstroms so eingestellt wird, dass Unterschiede in den Polarisationsunderstenden der Elektroden ausgeglichen werden.

10. Verfahren nach Anspruch 9, **dadurch gekennzeichnet, dass** als zweite Gaskomponente Stickoxide bestimmt werden.

11. Verfahren nach Anspruch 9, **dadurch gekennzeichnet, dass** an die Elektroden (31, 33,33) beider elektrochemischer Konzentrationszellen ein konstanter Strom angelegt wird, wobei die Höhe der beiden Ströme verschieden ist.

## Claims

1. Gas sensor for determining gas components in gas mixtures, in particular NOx in the exhaust gases of internal combustion engines, comprising a sensor element (10) with a first inner gas space (13, 21) and two further inner gas spaces (15, 17), the further inner gas spaces (15, 17) extending in each case in the form of channels from the first inner gas space (13, 21), with a first electrochemical concentration cell for determining free oxygen in the gas mixture, which has a first electrode (31), which is located in one of the further inner gas spaces (15), and has a second electrode, with a second electrochemical concentration cell for determining the sum of free oxygen and oxygen resulting from or consumed in the decomposition of the gas component to be determined, which has a further first electrode (33), which is located in the other of the further inner gas spaces (17), as well as a further second, catalytically active electrode, the first electrode (31) of the first concentration cell being preceded in the direction of flow of the gas mixture by a diffusion resistance, which is substantially equal to a diffusion resistance preceding the first electrode (33) of the second concentration cell, **characterized in that** the gas sensor comprises a means for forming the difference between the potentiometric signals of the first and second concentration cells as well as a further means for applying a constant current to the electrodes of at least one of the electrochemical concentration cells, the constant current being set such that differences in the polarization resistances of the potentiometric electrodes are balanced out.

2. Gas sensor according to Claim 1, **characterized in that** a gas inlet opening (21) for the gas mixture into the interior of the sensor element is provided, and **in that** the first electrodes (31, 33) are arranged in a layer plane (11 b) of the sensor element away from the gas inlet opening (21) by a substantially equal diffusion distance.

3. Gas sensor according to Claim 1 or 2, **characterized in that** the further inner gas spaces (15, 17) are arranged parallel next to one another in a layer plane (11 b).

4. Gas sensor according to Claim 3, **characterized in that** the first inner gas space (13), in which an inner pumping electrode (28) of an electrochemical pumping cell is located, is formed between the gas inlet opening (21) and the further inner gas spaces (15, 17) in the direction of flow.

5. Gas sensor according to Claim 4, **characterized in that** a constant voltage is applied to the electrodes (28, 29) of the electrochemical pumping cell.

6. Gas sensor according to Claim 4 or 5, **characterized in that** an outer pumping electrode (29) of the electrochemical pumping cell is arranged in an inner gas space (19) in contact with reference gas.

7. Gas sensor according to one of the preceding claims, **characterized in that** the second electrode (35) is exposed to the gas mixture.

8. Gas sensor according to one of the preceding claims, **characterized in that** both concentration cells are spatially arranged in such a way that both are exposed to comparable temperature conditions.

9. Method for determining gas components in gas mixtures with a gas sensor according to one of the preceding claims, the concentration of free oxygen being determined by means of the first electrochemical concentration cell of the sensor element contained in the gas sensor and the sum of free oxygen and at least one second gas component being determined by means of the second electrochemical concentration cell of the sensor element, **characterized in that** a measuring signal is formed by the difference between the potentiometric signals of the two electrochemical concentration cells and **in that** a constant current is applied to the electrodes of at least one of the electrochemical concentration cells, the size of the constant current being set such that differences in the polarization resistances of the electrodes are balanced out.

10. Method according to Claim 9, **characterized in that** nitrogen oxides are determined as the second gas component.

11. Method according to Claim 9, **characterized in that** a constant current is applied to the electrodes (31, 33, 35) of the two electrochemical concentration cells, the level of the two currents being different.

## Revendications

1. Détecteur de gaz destiné à déterminer des composants gazeux dans des mélanges de gaz et en particulier de NOx dans les gaz d'échappement de moteurs à combustion interne, et contenant
un élément de détection (10) qui présente pour le gaz un premier espace interne (13, 21) et deux autres espaces internes (15, 17), les autres espaces internes (15, 17) pour le gaz partant en forme de canal du premier espace interne (13, 21) pour le gaz,
une première cellule électrochimique de concentration destinée à déterminer l'oxygène libre dans le mélange de gaz et présentant une première électrode (31) située dans un des autres espaces internes (15) pour le gaz ainsi qu'une deuxième électrode,
une deuxième cellule électrochimique de concentration destinée à déterminer la somme de l'oxygène libre et de l'oxygène résultant de la décomposition des composants de gaz à déterminer ou consommés à cette occasion, et qui présente une autre première électrode (33) qui est située dans l'autre espace interne (17) pour le gaz ainsi qu'une autre deuxième électrode catalytiquement active,
une barrière à la diffusion étant placée en amont de la première électrode (31) de la première cellule de concentration dans la direction d'écoulement du mélange de gaz et étant essentiellement identique à une barrière à la diffusion placée en amont de la première électrode (33) de la deuxième cellule de concentration, **caractérisé en ce que**
le détecteur de gaz comprend un moyen qui forme la différence entre les signaux potentiométriques de la première et de la deuxième cellule de concentration ainsi qu'un autre moyen qui applique des courants constants sur les électrodes d'au moins une des cellules électrochimiques de concentration et
**en ce que** les courants constants sont réglés de manière à compenser les différences entre les résistances de polarisation des électrodes potentiométriques.

2. Détecteur de gaz selon la revendication 1, **caractérisé en ce qu'**il présente une ouverture (21) d'entrée du mélange de gaz dans l'élément de détection et **en ce que** les premières électrodes (31, 33) sont séparées essentiellement par un même parcours de diffusion de l'ouverture (21) d'entrée de gaz dans un plan de couche (11 b) de l'élément de détection.

3. Détecteur de gaz selon les revendications 1 ou 2, **caractérisé en ce que** les autres espaces internes (15, 17) pour le gaz sont disposés parallèlement et l'un à côté de l'autre dans un plan de couche (11 b).

4. Détecteur de gaz selon la revendication 3, **caractérisé en ce que** le premier espace interne (13) pour le gaz dans lequel est située une électrode interne de pompage (28) d'une cellule électrochimique de pompage est formé entre l'ouverture (21) d'entrée de gaz et les autres espaces internes (15, 17) pour le gaz dans la direction d'écoulement du mélange de gaz.

5. Détecteur de gaz selon la revendication 4, **caractérisé en ce qu'**une tension constante est appliquée sur les électrodes (28, 29) de la cellule électrochimique de pompage.

6. Détecteur de gaz selon les revendications 4 ou 5, **caractérisé en ce qu'**une électrode extérieure de pompage (29) de la cellule électrochimique de pompage est disposée dans un espace interne (19) pour le gaz en contact avec un gaz de référence.

7. Détecteur de gaz selon l'une des revendications précédentes, **caractérisé en ce que** la deuxième électrode (35) est exposée au mélange de gaz.

8. Détecteur de gaz selon l'une des revendications précédentes, **caractérisé en ce que** les deux cellules de concentration sont disposées spatialement de manière à être exposées à des conditions de température similaires.

9. Procédé de détermination de composants gazeux de mélanges de gaz à l'aide d'un détecteur de gaz selon l'une des revendications précédentes, dans lequel
la concentration de l'oxygène libre est déterminée au moyen de la première cellule électrochimique de concentration de l'élément de détection que contient le détecteur de gaz et la somme de l'oxygène libre et d'au moins un deuxième composant du gaz est déterminée au moyen de la deuxième cellule électrochimique de concentration de l'élément de détection,
**caractérisé en ce que**
un signal de mesure est formé par la différence entre les signaux potentiométriques des deux cellules électrochimiques de concentration et
**en ce que** des courants constants sont appliqués sur les électrodes d'au moins une des cellules électrochimiques de concentration, les niveaux des courants constants étant réglés de manière à compenser les différences entre les résistances de polarisation des électrodes.

10. Procédé selon la revendication 9, **caractérisé en ce que** les oxydes d'azote sont déterminés comme deuxième composant du gaz.

11. Procédé selon la revendication 9, **caractérisé en ce que** des courants constants sont appliqués sur les électrodes (31, 33, 35) des deux cellules électrochimiques de concentration et **en ce que** les deux courants ont des niveaux différents.
